# EUROPEAN PATENT SPECIFICATION

(11) **EP 1 370 552 B1**
(45) Date of publication and mention of the grant of the patent: **14.02.2007**
(21) Application number: 02713884.1
(22) Date of filing: 22.03.2002
(51) Int. Cl.: C07D 403/04, C07D 401/14, A61K 31/517, A61P 15/10, C07D 403/12, A61P 35/00

(54) **RHO-KINASE INHIBITORS**
RHO-KINASE INHIBITOREN
INHIBITEURS DE RHO-KINASE

(30) Priority: 23.03.2001 US 277974 P; 29.08.2001 US 315338 P
(43) Date of publication of application: 17.12.2003
(73) Proprietor: Bayer Pharmaceuticals Corporation, West Haven, CT 06516 (US)
(72) Inventor: NAGARATHNAM, Dhanapalan, Bethany, CT 06524 (US); WANG, Chunguang, Hamden, CT 06514 (US)
(74) Representative: Portal, Gérard
(86) International application number: PCT/US2002/008660
(87) International publication number: WO 2002/076977

(56) References cited:
- EP-A- 0 602 851
- WO-A-95/15758
- WO-A-96/39145
- WO-A-97/03069
- WO-A-99/35132

## Description

### Field of the Invention

This application claims the benefit of the filing date of U.S. Provisional Application Serial No. 60/277,974, filed March 23, 2001 and U.S. Provisional Application Serial No.: 60/315,338, filed August 29, 2001.

The present invention relates to compounds and derivatives thereof, their synthesis, and their use as Rho-kinase inhibitors. These compounds of the present invention are useful for inhibiting tumor growth, treating erectile dysfunction, and treating other indications mediated by Rho-Kinase, *e.g*., coronary heart disease.

### Background

The pathology of a number of human and animal diseases including hypertension, erectile dysfunction, coronary cerebral circulatory impairments, neurodegenerative disorders and cancer can be linked directly to changes in the actin cytoskeleton. These diseases pose a serious unmet medical need. The actin cytoskeleton is composed of a meshwork of actin filaments and actin-binding proteins found in all eukaryotic cells. In smooth muscle cells the assembly and disassembly of the actin cytoskeleton is the primary motor force responsible for smooth muscle contraction and relaxation. In non-muscle cells, dynamic rearrangements of the actin cytoskeleton are responsible for regulating cell morphology, cell motility, actin stress fiber formation, cell adhesion and specialized cellular functions such as neurite retraction, phagocytosis or cytokinesis (Van Aelst, et al. *Genes Dev* **1997**, *11*, 2295).

The actin cytoskeleton is controlled by a family of proteins that are a subset of the Ras superfamily of GTPases. This subset currently consists of RhoA through E and RhoG (refereed to collectively as Rho), Rac 1 and 2, Cdc42Hs and G25K and TC10 isoforms (Mackay, et al. *J Biol Chem* **1998,** *273*, 20685). These proteins are GTP (guanine nucleotide triphosphate) binding proteins with intrinsic GTPase activity. They act as molecular switches and cycles between inactive GDP (guanine nucleotide diphosphate) bound and active GTP bound states. Using biochemical and genetic manipulations, it has been possible to assign functions to each family member. Upon activation the Rho proteins controls the formation of actin stress fibers, thick bundles of actin filaments, and the clustering of integrins at focal adhesion complexes. When activated the Rac proteins control the formation of lamellopodia or membrane ruffles on the cell surface and Cdc42 controls filopodia formation. Together this family of proteins plays a critical part in the control of key cellular functions including cell movement, axonal guidance, cytokinesis, and changes in cell morphology, shape and polarity.

Depending on the cell type and the activating receptor, the Rho proteins can control different biological responses. In smooth muscle cells, Rho proteins are responsible for the calcium sensitization during smooth muscle contraction. In non-smooth muscle cells the Rho GTPases are responsible for the cellular responses to agonist such as lysophosphatidic acid (LPA), thrombin and thromboxane A₂ (Fukata, et al. *Trends Pharcol Sci* **2001,** *22*, 32). Agonist response is coupled through heterotrimeric G proteins Gₐₗₚₕₐ₁₂ or Gₐₗₚₕₐ₁₃ (Goetzl, et al. *Cancer Res* **1999,** *59,* 4732; Buhl, et al. *J Biol Chem* **1995,** *270, 24631)* though other receptors may be involved. Upon activation Rho GTPases activate a number of downstream effectors including PIP5-kinase, Rhothekin, Rhophilin, PKN and Rho kinase isoforms ROCK-1/ROKbeta and ROCK-1/ROKalpha (Mackay and Hall *J Biol Chem* **1998**, *273*, 20685; Aspenstrom *Curr Opin Cell Biol* **1999,** *11*, 95; Amano, et al. *Exp Cell Res* **2000,** *261, 44).*

Rho kinase was identified as a RhoA interacting protein isolated from bovine brain (Matsui, et al. *Embo J* **1996,** *15*, 2208). It is a member of the myotonic dystrophy family of protein kinase and contains a serine/threonine kinase domain at the amino terminus, a coiled-coil domain in the central region and a Rho interaction domain at the carboxy terminus (Amano, et al. *Exp Cell Res* **2000**, *261,* 44). Its kinase activity is enhanced upon binding to GTP-bound RhoA and when introduced into cells, it can reproduce many of the activities of activated RhoA. In smooth muscle cells Rho kinase mediates calcium sensitization and smooth muscle contraction and inhibition of Rho kinase blocks 5-HT and phenylephrine agonist induced muscle contraction. When introduced into non-smooth muscle cells, Rho kinase induces stress fiber formation and is required for the cellular transformation mediated by RhoA (Sahai, et al. *Curr Biol* **1999**, 9, 136). Rho kinase regulates a number of downstream proteins through phosphorylation, including myosin light chain (Somlyo, et al. *J Physiol (Lond)* **2000**, *522 Pt 2,* 177), the myosin light chain phosphatase binding subunit (Fukata, et al. *J Cell Biol* **1998**, *141,* 409) and LIM-kinase 2 (Sumi, et al. *J Bio Chem* **2001**, *276*, 670).

Inhibition of Rho kinase activity in animal models has demonstrated a number of benefits of Rho kinase inhibitors for the treatment of human diseases. Several patents have appeared claiming (+)-trans-4-(1-aminoethyl)-1-(pyridin-4-ylaminocarbonyl)cyclohexane dihydrochloride monohydrate (WO-00078351, WO-00057913) and substituted isoquinolinesulfonyl (EP-00187371) compounds as Rho kinase inhibitors with activity in animal models. Other patents (EP-A-0 602 851, WO95/15758, WO96/39145A, WO97/03069A, WO99/35132) disclose 2-non substituted quinazoline derivatives as Rho-kinase inhibitors. These include models of cardiovascular diseases such as hypertension (Uehata, et al. *Nature* **1997**, *389*, 990), atherosclerosis (Retzer, et al. *FEBS Lett* **2000**, *466,* 70), restenosis (Eto, et al. *Am J Physiol Heart Circ Physiol* **2000**, *278*, H1744; Negoro, et al. *Biochem Biophys Res Commun* **1999***, 262,* 211), cerebral ischemia (Uehata, et al. *Nature* **1997,** *389*, 990; Seasholtz, et al. *Circ Res* **1999,** *84,* 1186; Hitomi, et al. *Life Sci* **2000**, *67,* 1929; Yamamoto, et al. *J Cardiovasc Pharmacol* **2000**, *35,* 203), cerebral vasospasm (Sato, et *al*. *Circ Res* **2000**, *87,* 195; Kirn, et al. *Neurosurgery* **2000**, *46,* 440), penile erectile dysfunction (Chitaley, et al*. Nat Med* 2001, 7, 119), central nervous system disorders such as neuronal degeneration and spinal cord injury (Hara, et al. *J Neurosurg* **2000***, 93, 94;* Toshima, et al. *Stroke* **2000**, *31*, 2245) and in neoplasias where inhibition of Rho kinase has been shown to inhibit tumor cell growth and metastasis (Itoh, et al. *Nat Med* **1999**, *5,* 221; Somlyo, et al. *Biochem Biophys Res Commun* **2000***, 269,* 652)*,* angio genesis (Uchida, et al. *Biochem Biophys Res Commun* **2000**, *269,* 633; Gingras, et al. *Biochem J* **2000**, 348 *Pt 2*, 273), arterial thrombotic disorders such as platelet aggregation (Klages, et al. *J Cell Biol* **1999**, *144*, 745; Retzer, et al. *Cell Signal* **2000**, 12, 645) and leukocyte aggregation (Kawaguchi, et al. *Eur J Phamacol* **2000**, *403,* 203; Sanchez-Madrid, et al. *Embo J* **1999***, 18,* 501)*,* asthma (Setoguchi, et al. *Br J Pharmacol* **2001***, 132,* 111; Nakahara, et al. *Eur J Pharmacol* **2000***, 389,* 103), regulation of intraoccular pressure (Honjo, et al. *Invest Ophthalmol Vis Sci* **2001**, *42*, 137) and bone resorption (Chellaiah, et al. *J Biol Chem* **2000**, *275*, 11993; Zhang, et al. *J Cell Sci* **1995**, *108*, 2285).

The inhibition of Rho kinase activity in patients has benefits for controlling cerebral vasospasms and ischemia following subarachnoid hemorrhage (*Pharma Japan* **1995**, *1470,* 16)*.*

### Summary of the Invention

The compounds and their derivatives presented in this invention are useful as Rho Kinase inhibitors and thus have utilities in the treatment of hypertension, atherosclerosis, restenosis, cerebral ischemia, cerebral vasospasm, neuronal degeneration, spinal cord injury, cancers of the breast, colon, prostate, ovaries, brain and lung and their metastases, thrombotic disorders, asthma, glaucoma and osteoporosis.
In addition, the compounds of the invention are useful to treat erectile dysfunction, i.e., erectile dysfunction mediated by Rho-kinase. Erectile dysfunction can be defined as an inability to obtain or sustain an erection adequate for intercourse, WO 94/28902, U.S.P. 6,103,765 and U.S.P. 6,124,461.

The invention provides compounds of formulae

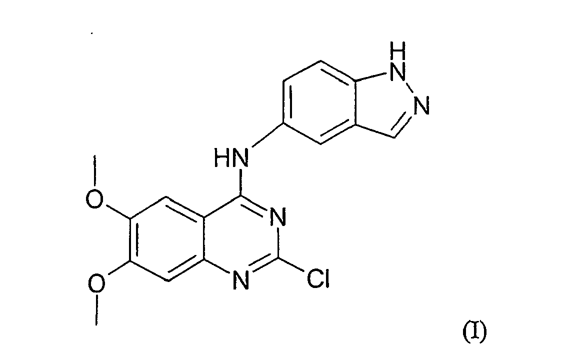

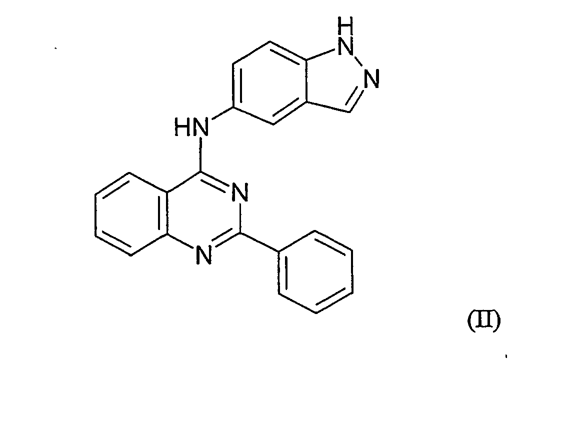

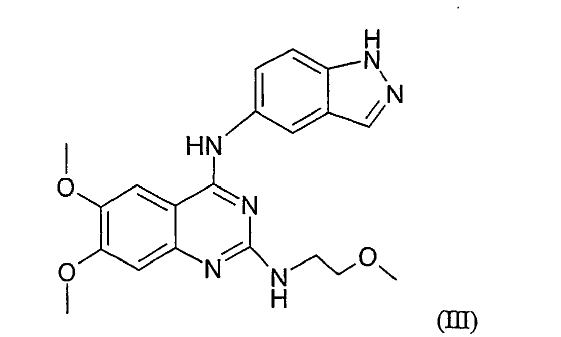

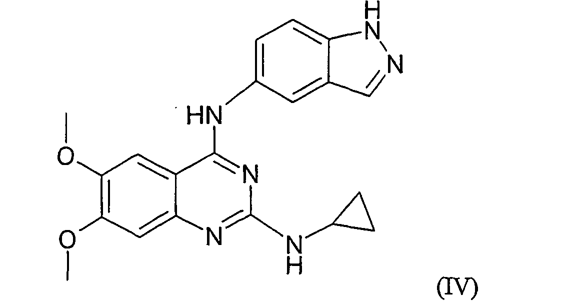

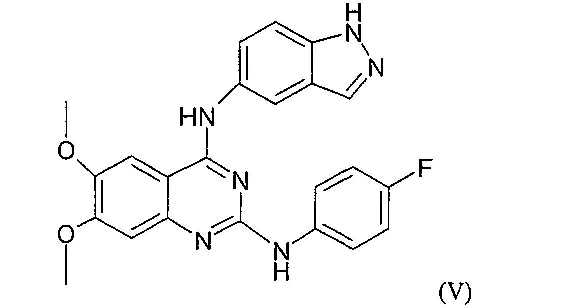

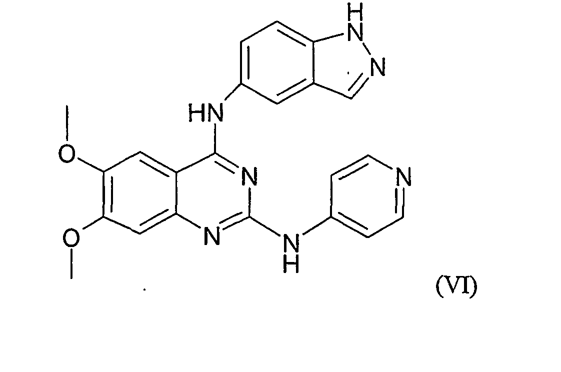

The present invention is also directed to pharmaceutically acceptable salts of Formulae I-VI. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, sulphonic acid, acetic acid, trifluoroacetic acid, malic acid, tartaric acid, citric acid, lactic acid, oxalic acid, succinic acid, fumaric acid, maleic acid, benzoic acid, salicyclic acid, phenylacetic acid, and mandelic acid. In addition, pharmaceutically acceptable salts include acid salts of inorganic bases, such as salts containing alkaline cations (e.g., Li⁺, Na⁺ or K⁺), alkaline earth cations (e.g., Mg⁺, Ca⁺ or Ba⁺), the ammonium cation, as well as acid salts of organic bases, including aliphatic and aromatic substituted ammonium, and quaternary ammonium cations, such as those arising from protonation or peralkylation of triethylamine, *N,N-*diethylamine, *N,N-*dicyclohexylamine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formulae I-VI possess asymmetric carbons and can therefore exist in racemic and optically active forms. Methods of separation of enantiomeric and diastereomeric mixtures are well known to one skilled in the art. The present invention encompasses any isolated racemic or optically active form of compounds described in Formulae I-VI which possess Rho-kinase inhibitory activity.

The invention also includes pharmaceutical compositions including a compound of Formulae I-VI, and a physiologically acceptable carrier.

The invention moreover encompasses treating indications mediated by Rho-kinase, by administering a compound of Formulae I-VI, or a pharmaceutical composition containing a compound of Formulae I-VI. Thus, the invention encompasses treating cardiovascular diseases such as hypertension, artherosclerosis, restenosis and cerebral ischemia, or vasospasm central nervous system disorders such as neuronal degeneration and spinal cord injury, erectile dysfunction, e.g., in patients who do not have satisfactory response to PDE-5 inhibitors, and cancer (e.g., tumor growth) mediated by Rho-kinase, by administering, e.g., to a host in need thereof, of an effective amount of a compound of Formulae I-VI. Cancers and tumors mediated by Rho-kinase include cancers of the breast, colon, prostate, ovaries, brain and lung and their metastases.

The compounds may be administered orally, topically, parenterally, by inhalation or spray, vaginally, rectally or sublingually in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Compounds of the invention may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix.

Pharmaceutical compositions of the invention may also be in the form of oil-in-water emulsions. The oil phase may be a vegetable oil, for example olive oil or arachis oil, or a mineral oil, for example, liquid paraffin or mixtures of these. Suitable emulsifying agents may be naturally-occurring gums, for example, gum acacia or gum tragacanth, naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal temperature or vaginal temperature and will therefore melt in the rectum or vagina to release the drug. Such materials include cocoa butter and polyethylene glycols.

Moreover, for treatment of erectile dysfunction, the present pharmaceutical compositions may take any form which is suitable for administration to the penis either via injection into the corpora cavemosa or transurethral administration, or topically applied to the urethral meatus. In the case of injection into the corpora cavernosa, the pharmaceutical composition is suitably in the form of a saline solution. Preferably, the pharmaceutical composition is in a form suitable for transurethral administration, and in this case the composition is typically in the form of a solution, an ointment, or a suppository. Typically, the pharmaceutical composition is administered 1 to 50 minutes, preferably 10 to 20 minutes, prior to the time of commencing sexual intercourse.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily vaginal dosage regime will preferably be from 0.01 to 200 mg/Kg of total body weight. The daily topical dosage regimen will preferably be from 0.01 to 200 mg administered between one to four times daily. The transdermal concentration will preferably be that required to maintain a daily dose is of from 0.1 to 200 mg/Kg. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses of a compound of Formula I or a pharmaceutically acceptable salt thereof given for a defined number of days, can be ascertained by those skilled in the art using conventional treatment tests.

The present compounds and compositions exhibit Rho-kinase inhibitory activity, and are thus useful to treat the indications listed above, *e.g*., indications mediated by Rho-kinase. By indications mediated by Rho-kinase is meant diseases or conditions whose progression proceeds, at least in part, via the Rho pathway.

Rho-kinase inhibitory activity, *e.g*., ROCK-1 inhibition, can be evaluated as follows:

The kinase domain of human ROCK-1, amino acids 27-530, is isolated as a glutathione S-transferase fusion protein from Sf9 insect cells. The protein is partially purified by glutathione Sepharose 4B (Pharmacia Biotech, Piscataway, NJ) affinity purification. Reactions is carried out in 96-well plates in a total volume of 100 uL containing 50 mM N-[2-Hydoryethyl]piperaxine-N'-[2-ethanesulfonic acid] pH 7.5, 5 mM MgCl₂, 1 mM dithiothreitol, 6 µM ATP, 0.2 µCi [³³P]ATP (NEN, Boston, MA), 1 µg myelin basic protein and 0.1 µg ROCK-1. Test compounds are dissolved in 100% dimethylsulfoxide, diluted to the appropriated concentration and added to the reaction. The final concentration of dimethylsulfoxide did not exceed 0.5%. The reaction is run for one hour at room temperature. The reaction is stopped with the addition of 7 mL of 1 N HCL, transferred to P30 membranes and the amount of [³³P]ATP, as counts per minute (c.p.m.) incorporated into the substrate, myelin basic protein, is read in a BetaPlate Reader (Packard Instrument Co., Meriden, CT.). (All reagents were purchased from Sigma Chemical Co., St. Louis, MO unless stated otherwise.) Percentage inhibition is measured by the amount of incorporation of radioactivity in the presence of the test compound when compared to the amount of incorporation in the absence of the test compound.

Inhibitory activity can also be evaluated by measurement of stress fiber formation, performed essentially as described by Ridley, A.J., and A. Hall, Cell 70:389-399 (1992). Human fibrosarcoma HT1080 (CCL-121, American Type Culture Collection, Manassas, VA) cells are plated on 22 X 22 mm #1 glass cover slips in six-well tissue culture plates (Costar) at 2.5 X 10⁴ cells/well in Delbeco's modified Eagle's Medium (DMEM, Gibco) supplemented with 10% fetal calf serum. Cells are maintained in a humidified, 5% CO₂ atmosphere at 37°C. After 24 hours the culture medium is removed and replaced with medium without 10% fetal calf serum and the cells cultured for an additional 48 hours. Test compounds are dissolved in 100% dimethylsulfoxide, diluted to the appropriated concentration and added to the culture medium 60 minutes prior to the induction of stress fiber formation. The final concentration of dimethylsulfoxide did not exceed 0.25%. Stress fiber formation is induced by the addition of lysophosphatidic acid (1-oleoyl-2-hydroxy-*sn*-glycerol-3-phosphate, Avanti Polar-Lipids, Alabaster, Al) to 10 µM final concentration in Delbeco's modified Eagle's Medium containing 0.1% fatty acid free bovine serum albumin for 15 minutes at 37°C. Cells are fixed with 4% paraformaldeyhde (Poly Scientific, Bay Shore, NJ) in phosphate buffered saline (PBS) for 15 minutes. Cells are then washed 3 times in PBS and them permeabilized using a solution containing 40mM piperazine-N-N'bis[2-ethanesulfonic acid], 50 mM N-[2-hydoryethyl]piperaxine-N'-[2-ethanesulfonic acid], 0.1% Triton X-100, 75 mM NaCI, mM MgCl₂, 0.5 mM EGTA, pH 7.2 for 2 minutes at room temperature. The cells are washed 3 times for 5 minutes each in PBS and then actin stress fibers are stained using 10 units/mL rhodamine phalloidin (Molecular Probes, Eugene, OR) in PBS for 60 minutes at room temperature. The cells are washed 3 times with PBS and the cover slips mounted on glass microscope slides. The percentage of stress fiber positive cells on each slide was determined visually using a Nikon Labphoto-2 microscope. At least 100 cells were counted per slide and experiments were done in duplicate. Percentage inhibition is measured by counting the number of stress fiber positive cells in the presence of the test compound when compared to the number of stress fiber positive cells in the absence of the test compound.

Using the above protocols, all of the compounds as disclosed herein are determined to have Rho-kinase inhibitory activity.

The compounds of the invention can be made according to routine, conventional chemical methods, and/or as disclosed below, from starting materials which are either commercially available or produceable according to routine, conventional chemical methods. General methods for the preparation of the compounds are given below, and the preparation of representative compounds is specifically illustrated in the Examples.

### ABBREVIATIONS AND ACRONYMS

When the following abbreviations are used herein, they have the following meaning:
- Ac₂O: acetic anhydride
- anhy: anhydrous
- *n*-BuOH: *n*-butanol
- *t*-BuOH: *t*-butanol
- CD₃OD: methanol-*d₄*
- Celite^{®}: diatomaceous earth filter agent, ® Celite Corp.
- CH₂Cl₂: methylene chloride
- CI-MS: chemical ionization mass spectroscopy
- conc: concentrated
- dec: decomposition
- DME: dimethoxyethane
- DMF: *N,N* dimethylformamide
- DMSO: dimethylsulfoxide
- ELSD: evaporative light scattering detector
- EtOAc: ethyl acetate
- EtOH: ethanol (100%)
- Et₂O: diethyl ether
- Et₃N: triethylamine
- HPLC ES-MS: high performance liquid chromatography-electrospray mass spectroscopy
- NMM: 4-methylmorpholine
- Ph₃P: triphenylphosphine
- Pd(dppf)Cl₂: [1,1'-bis(diphenylphosphino)ferrocene]dichloropalladium(II)
- Pd(PPh₃)₄: tetrakis(triphenylphosphine)palladium(0)
- Pd(OAc)₂: palladium acetate
- P(O)Cl₃: phosphorous oxychloride
- RT: retention time (HPLC0
- rt: room temperature
- THF: tetrahydrofuran
- TFA: trifluoroacetic acid
- TLC: thin layer chromatography

### General Methods of Preparation

In the formulas used to describe the following general methods, R¹ and R² is hydrogen or methoxy, and R³ is methoxyethyl, cyclopropyl, 4-fluorophenyl or 4-pyridyl, appropriately selected in order to prepare the compounds I-VI of the invention.

### General Method A

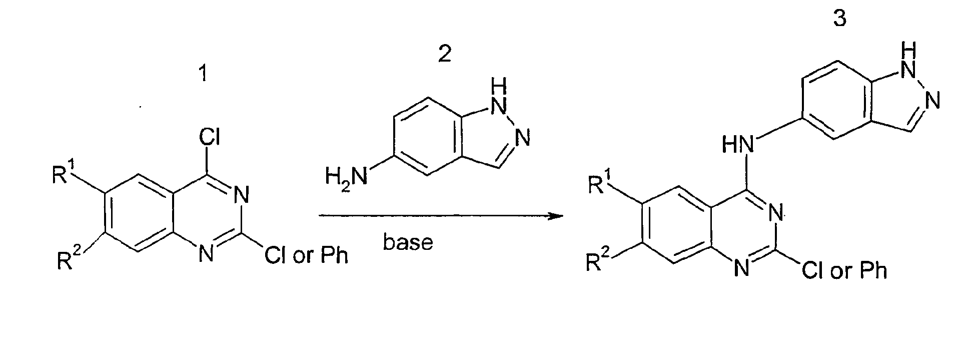

A mixture of compounds 1 and 2, and potassium acetate in THF/water is stirred at room temperature overnight. Water is added to the mixture resulting in the formation of a precipitate. The precipitate is washed with water, filtered, and dried under high vacuum to afford 3.

### General Method B

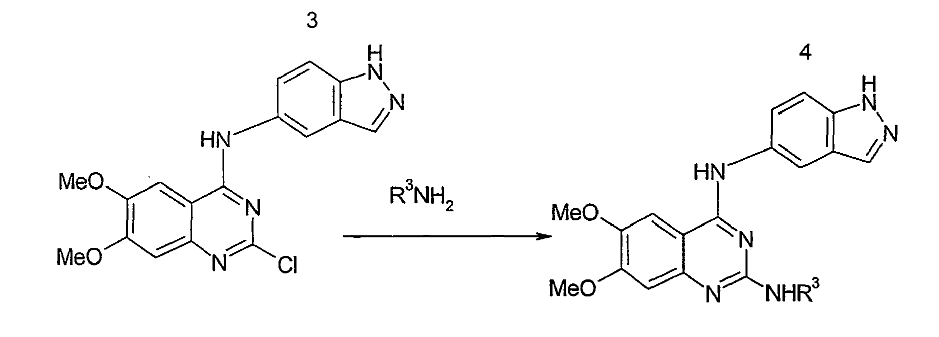

A mixture of compound 3, and a substituted amine or aniline is heated to 140°C for 2 hours. The mixture is cooled to room temperature and is treated with ether to form precipitate or purified by silica gel column chromatography. Purification of precipitate: The precipitate is filtered, washed with ether several times, and is dried under high vacuum to provide product.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius; and, unless otherwise indicated, all parts and percentages are by weight.

The entire disclosure of all applications, patents and publications, cited above or below, U.S. Patent Application No. 60/277,974 filed March 23, 2001 and U.S. Patent Application No. Serial No.: 60/315,338, filed August 29, 2001, are hereby incorporated by reference.

### Example 1

### Preparation of 4-N-5'-aminoindazole-2-chloro-6,7-dimethoxyquinazoline

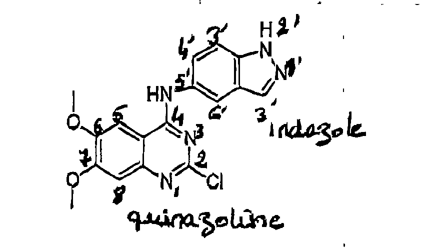

A mixture of 2,4-dichloro-6,7-dimethoxyquinazoline from step I (Aldrich Chemical Co., 226 g, 0.874 mol), 5-aminoindazole (130 g, 0.98 mol), and potassium acetate (111.5 g, 1.14 mol) in THF/water (2 L/0.9 L) is stirred at room temperature overnight. Water (2 L) is added to the mixture resulting in the formation of a precipitate. The precipitate is washed with water, filtered, and dried under high vacuum to afford product as a gray powder.

### Example 2

### Preparation of N-[2-(2,4-dichlorophenyl)-4-quinazolin]-N-(1H-indazol-5-yl)amine

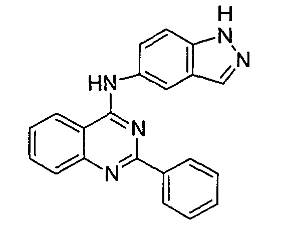

A mixture of 4-chloro-2-phenylquinazoline (Aldrich Chemical Co., 7.2 g, 30 mmol) and 5-aminoindazole (3.99 g, 30 mmol) in butanol (50 mL) is heated to 100°C overnight. After removal of solvent in vacuo the crude product is purified by silica gel column chromatography (gradient from 20% to 80% ethyl acetate/ hexane) to afford Example **2** (3.6 g). HPLC/MS: (M+H)⁺ 338 *m*/*z.* Retention time (HPLC/MS)= 3.65 min.

### General Synthetic Route to Examples 3-6

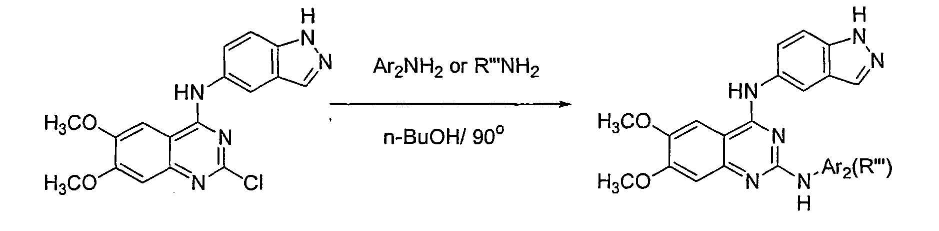

### Example 3

### Preparation of N2-(3-fluorophenyl)-N4-(1H-indazol-5-yl)-6,7-dimethoxy-2,4-quinazolinediamine

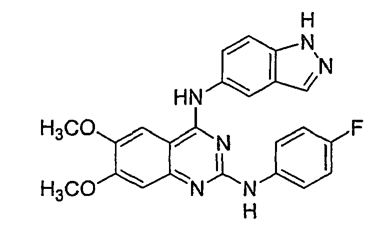

A suspension of 2-chloro-N-(1H-indazol-5-yl)-6,7-dimethoxy-4-quinazolinamine (0.1mmol) and 4-fluoroaniline (0.3 mmol) in n-butanol (1 mL) is shaken at 90°C for 72 h. The solvent is evaporated off and the residue is purified by HPLC to afford pure product. (M+H)⁺=431, RT (LC-MS)=2.92.

Using the method described above for Example **3**, and substituting the appropriate starting materials, Examples **4**-**6** were similarly prepared and are summarized below in Table 1.

**Table 1**

| | | | |
|---|---|---|---|
| 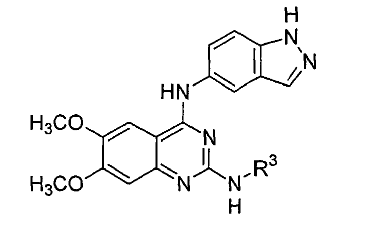 | | | |

| **Ex. No** | **R³** | **LC-MS RT (min)** | **Mass Spec (M+H)⁺** |
|---|---|---|---|
| **4** | 4-pyridinyl | 2.81 | 414 |
| **5** | 2-methoxyethyl | 2.74 | 395 |
| **6** | cyclopropyl | 2.78 | 377 |

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims

1. A compound of formulae I-VI 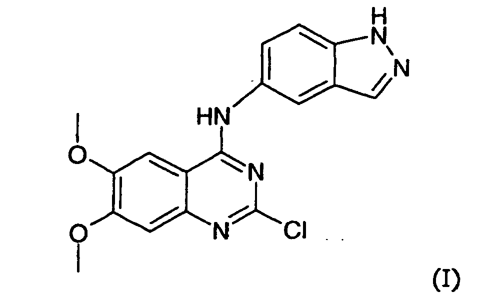 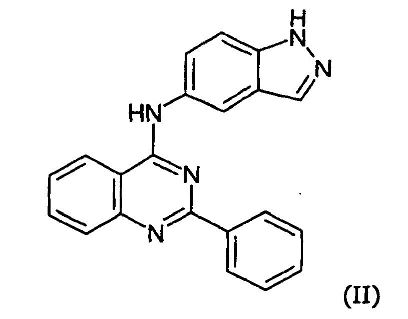 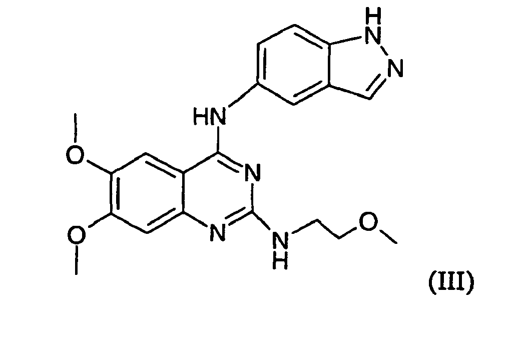 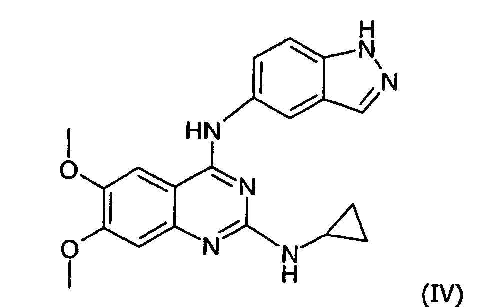 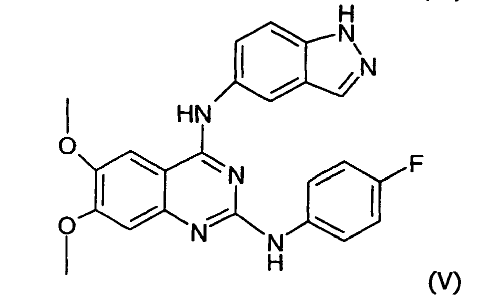 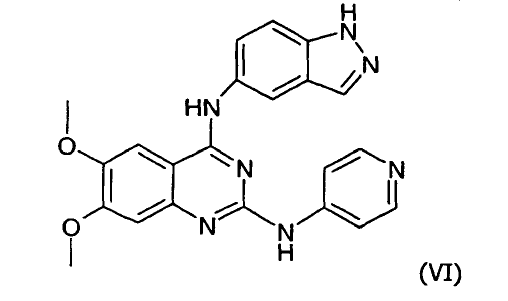 or a pharmaceutically acceptable salt thereof.

2. A compound of claim 1, of formula I

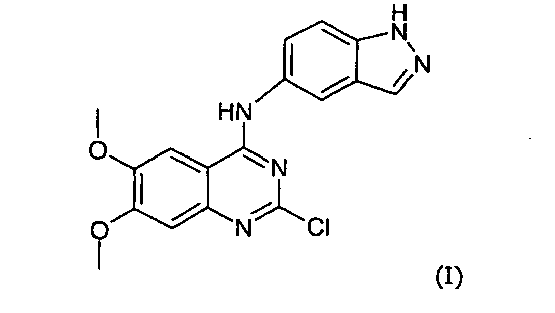

3. A compound of claim 1, of formula II

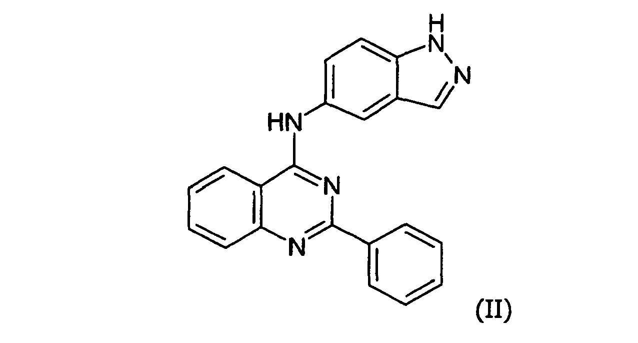

4. A compound of claim 1, of formula III

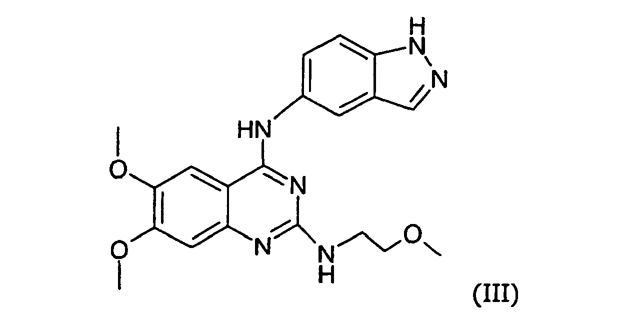

5. A compound of claim 1, of formula IV

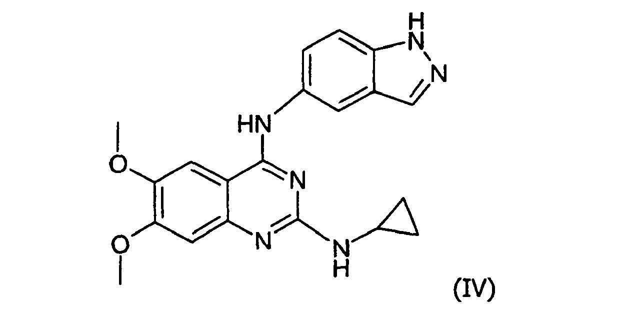

6. A compound of claim 1, of formula V

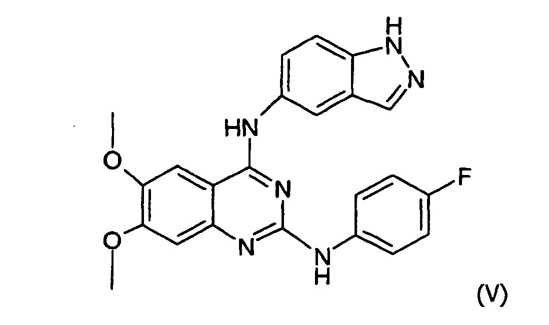

7. A compound of claim 1, of formula VI

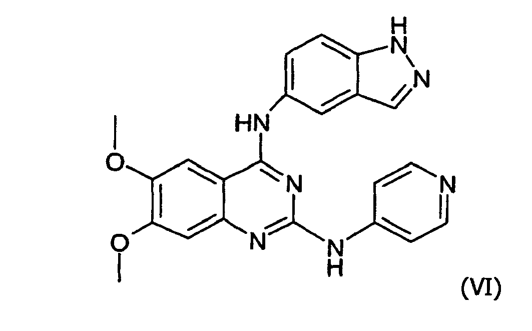

8. A method for the preparation of a compound as to claim 1, comprising (a) reacting compound of formula 1 with a compound of formula 2, in the presence of a base, to produce a compound of formula 3 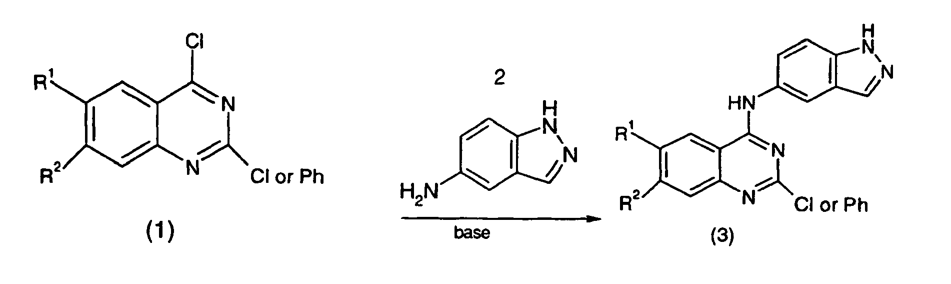 wherein R¹ and R² can independently be hydrogen or CH₃O-, and Ph is phenyl, and
(b) optionally, reacting a compound of formula 3 with R³NH₂ or Ar₂NH₂ to produce a compound of formula 4 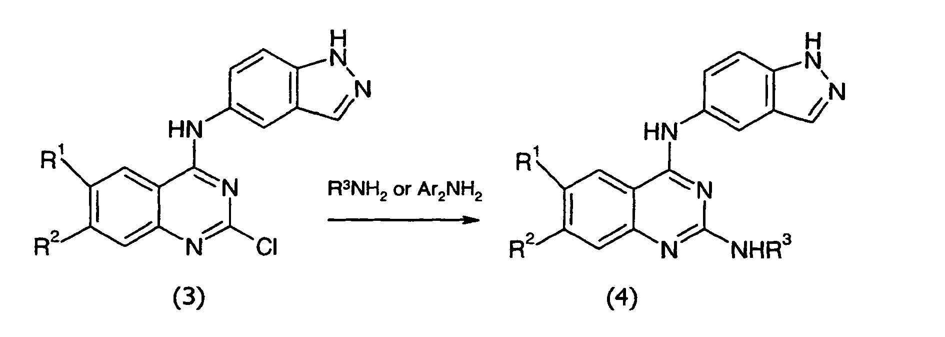 wherein R³ is CH₃O-CH₂CH₂-, or cyclopropyl, and Ar₂ is 4-fluorophenyl or pyridyl.

9. A compound according to formula (3) : 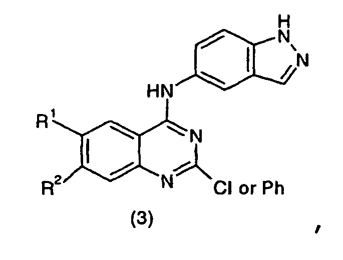 wherein R¹ and R² can independently be hydrogen or CH₃O-, and Ph is phenyl, or
formula (4) : 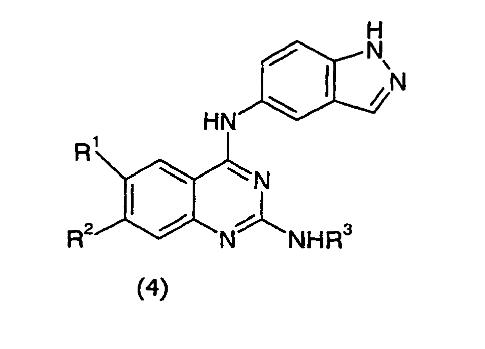 wherein R³ is CH₃O-CH₂CH₂-, or cyclopropyl, and Ar₂ is 4-fluorophenyl or pyridyl, **characterised in that** it is obtainable according to a method according to claim 8.

10. A pharmaceutical composition, **characterised in that** includes a compound according to any one of claims 1 to 7 or 9, and a physiologically acceptable carrier.

11. Use of a compound according to any one of claims 1 to 7 or 9 for the preparation of a medicament for treating an indication mediated by Rho-kinase.

12. Use of a compound according to any one of claims 1 to 7 or 9 for the preparation of a medicament for treating hypertension, atherosclerosis, restenosis, cerebral ischemia, cerebral vasospasm, neuronal degeneration, spinal cord injury, cancer of the breast, colon, prostate, ovaries, brain or lung, thrombotic disorders, asthma, glaucoma, osteoporosis or erectile dysfunction.

13. Use according to claim 11 or 12, wherein the host is a human.

## Patentansprüche

1. Zusammensetzung gemäß Formel I-VI 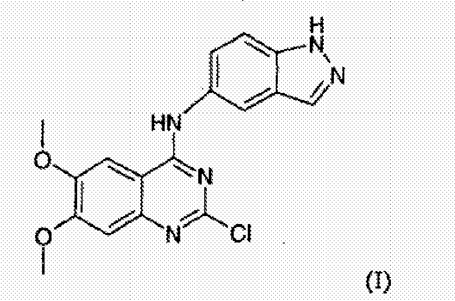 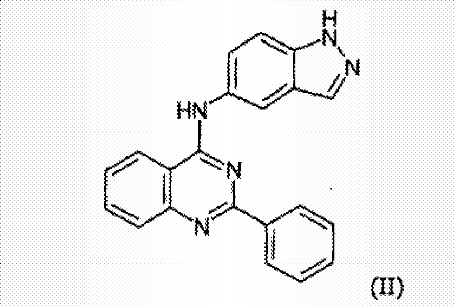 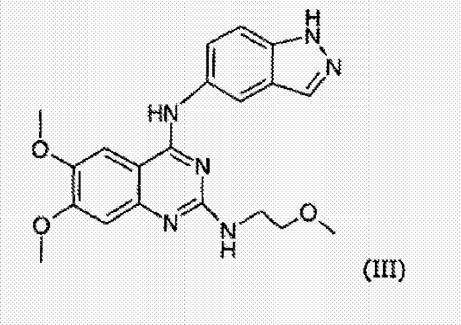 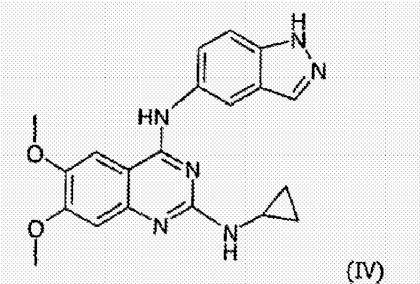 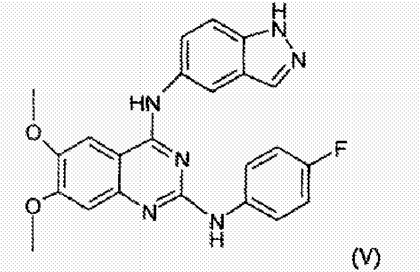 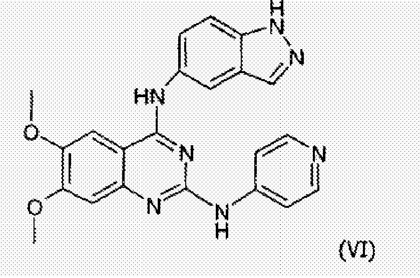 oder ein pharmazeutisch akzeptables Salz hiervon.

2. Zusammensetzung gemäß Anspruch 1 von Formel I

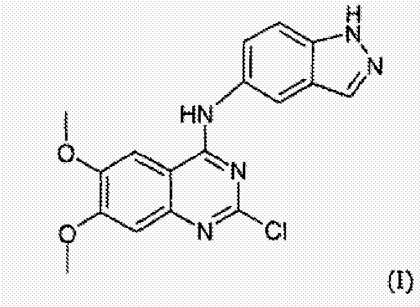

3. Zusammensetzung nach Anspruch 1 von Formel II

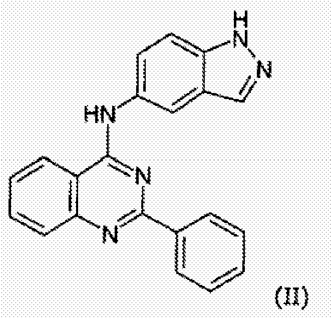

4. Zusammensetzung nach Anspruch 1 von Formel III

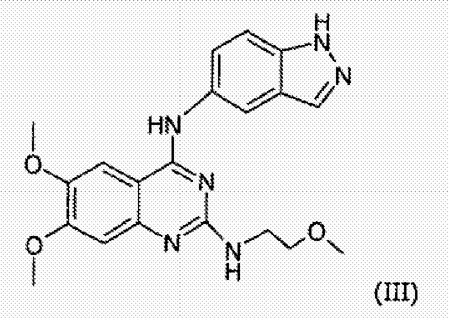

5. Zusammensetzung nach Anspruch 1 von Formel IV

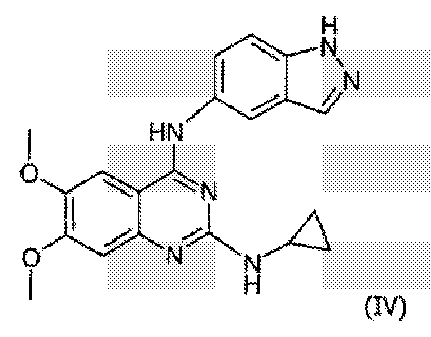

6. Zusammensetzung nach Anspruch 1 von Formel V

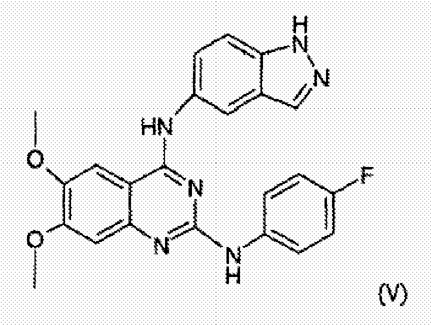

7. Zusammensetzung nach Anspruch 1 von Formel VI

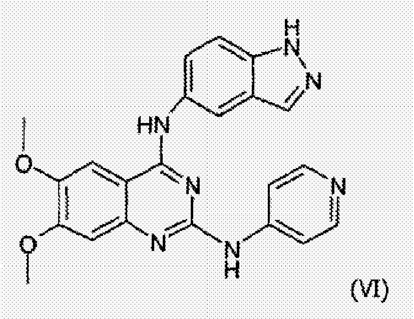

8. Verfahren zum Zubereiten einer Zusammensetzung gemäß Anspruch 1, umfassend
(a) Reagierenlassen von Zusammensetzung von Formel 1 mit einer Zusammensetzung von Formel 2, in Gegenwart einer Base, um eine Zusammensetzung von Formel 3 herzustellen 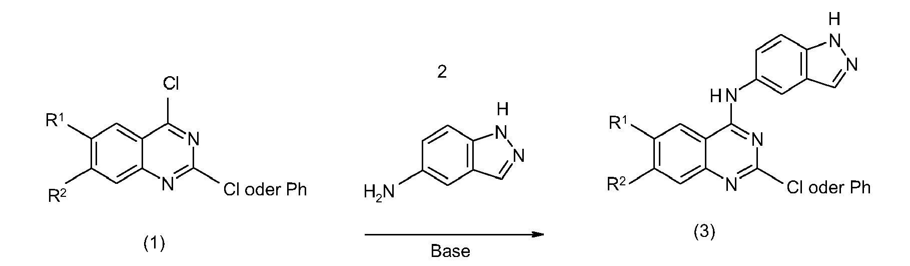 worin R¹ und R² unabhängig voneinander Wasserstoff oder CH₃O- sein können und Ph Phenyl ist, und
(b) optional Reagierenlassen einer Zusammensetzung von Formel 3 mit R³NH₂ oder Ar₂NH₂, um eine Zusammensetzung von Formel 4 herzustellen 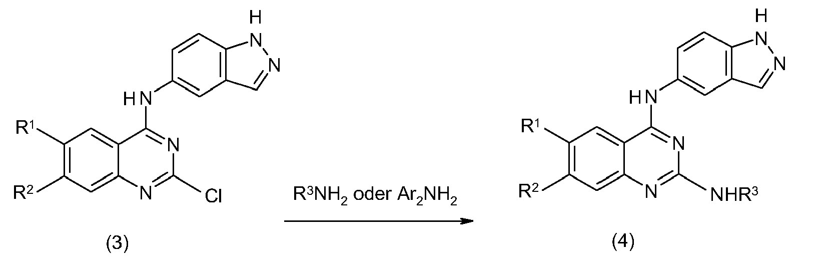
worin R³ CH₃O-CH₂CH₂- oder Cyclopropyl ist, und Ar₂ 4-Fluorophenyl oder Pyridyl ist.

9. Zusammensetzung entsprechend Formel (3): 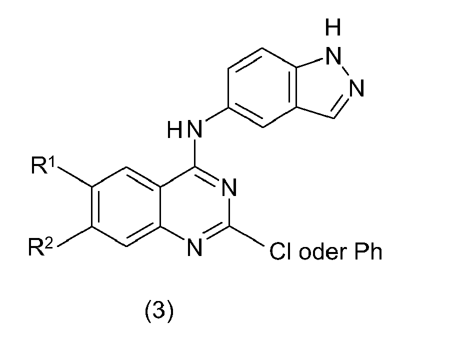 worin R¹ und R² unabhängig voneinander Wasserstoff oder CH₃O- sein können und Ph Phenyl ist,
oder
Formel (4): 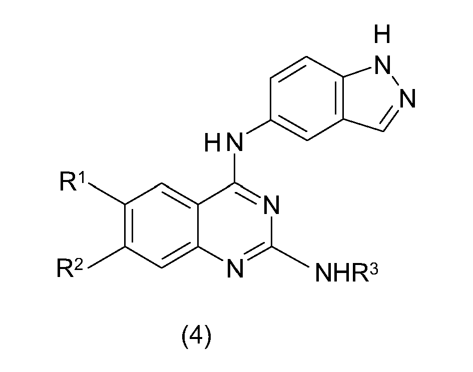 worin R³ CH₃O-CH₂CH₂CH₂-, oder Cyclopropyl ist, und Ar₂ 4-Fluorophenyl oder Pyridyl ist, **dadurch gekennzeichnet, daß** sie erhältlich ist entsprechend einem Verfahren gemäß Anspruch 8.

10. Pharmazeutische Zusammensetzung, **dadurch** charakterisiert, daß sie eine Zusammensetzung nach einem der Ansprüche 1 bis 7 oder 9 und einen physiologisch akzeptablen Träger enthält.

11. Verwendung einer Zusammensetzung nach einem der Ansprüche 1 bis 7 oder 9 zur Zubereitung eines Medikaments zur Behandlung einer durch Rho-kinase vermittelten Indikation.

12. Verwendung einer Zusammensetzung nach einem der Ansprüche 1 bis 7 oder 9 zur Zubereitung eines Medikaments zur Behandlung von Bluthochdruck, Arteriosklerose, Restenose, zerebrale Ischämie, zerebrales Vasospasma, neuronale Degeneration, Rückenmarksverletzung, Brustkrebs, Dickdarmkrebs, Prostatakrebs, Eierstockkrebs, Hirntumoren oder Lungenkrebs, thrombotischen Funktionsstörungen, Asthma, grüner Star, Osteoporose oder Erektionsstörungen.

13. Verwendung entsprechend Anspruch 11 oder 12, wobei der Wirt ein Mensch ist.

## Revendications

1. Composé de formules I à VI 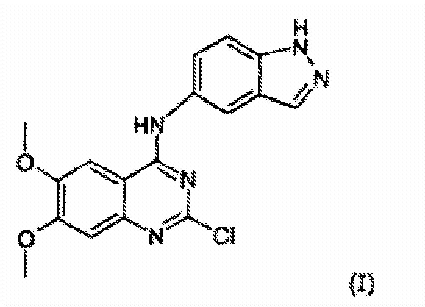 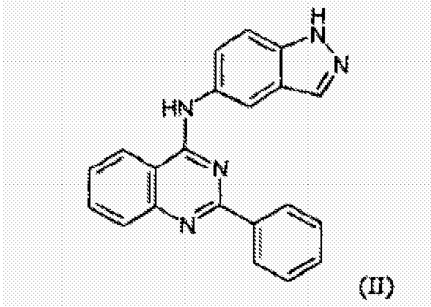 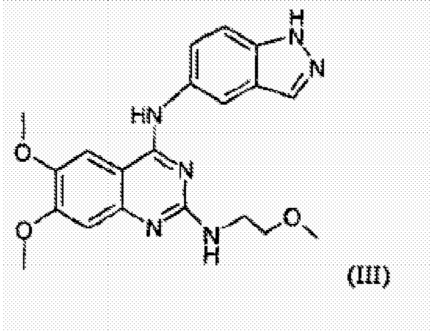 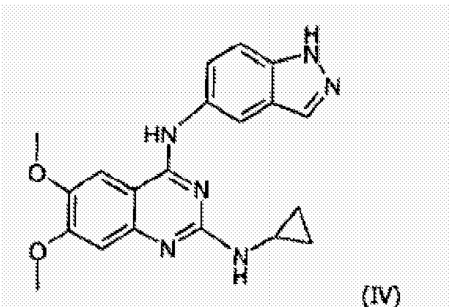 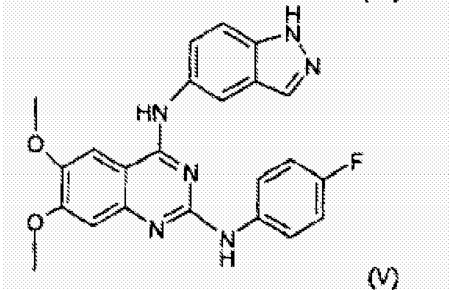 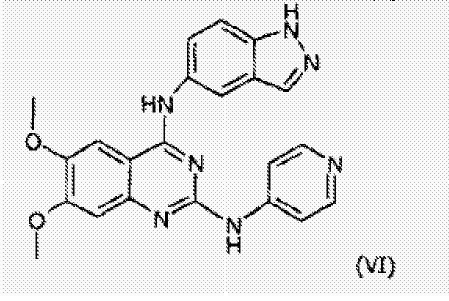 ou un sel pharmaceutiquement acceptable de celui-ci.

2. Composé selon la revendication 1, de formule I

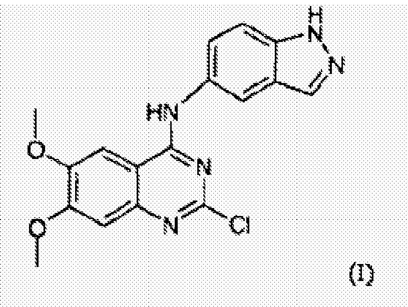

3. Composé selon la revendication 1, de formule II

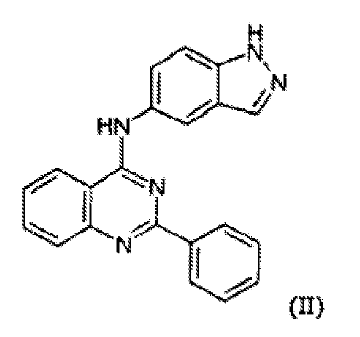

4. Composé selon la revendication 1, de formule III

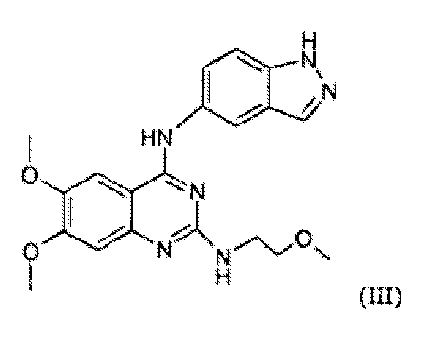

5. Composé selon la revendication 1, de formule IV

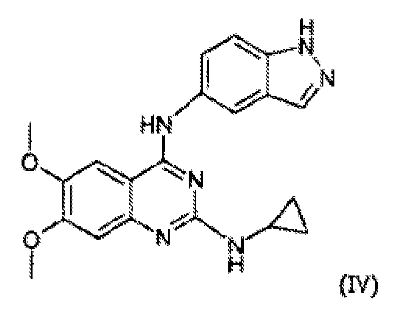

6. Composé selon la revendication 1, de formule V

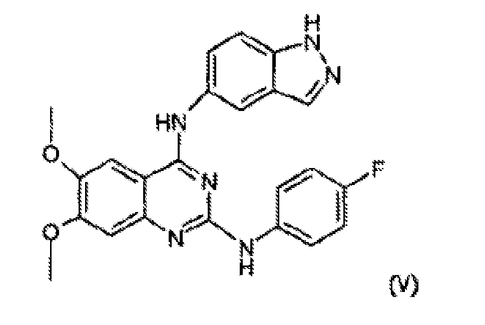

7. Composé selon la revendication 1, de formule VI

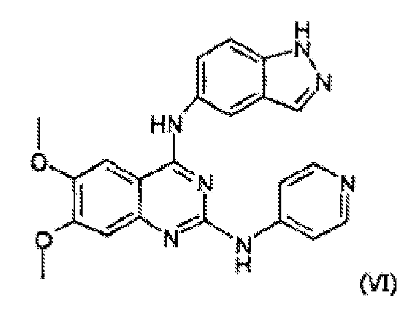

8. Procédé de préparation d'un composé selon la revendication 1, comprenant les étapes consistant à :
a) faire réagir un composé de formule 1 avec un composé de formule 2, en présence d'une base, pour produire un composé de formule 3 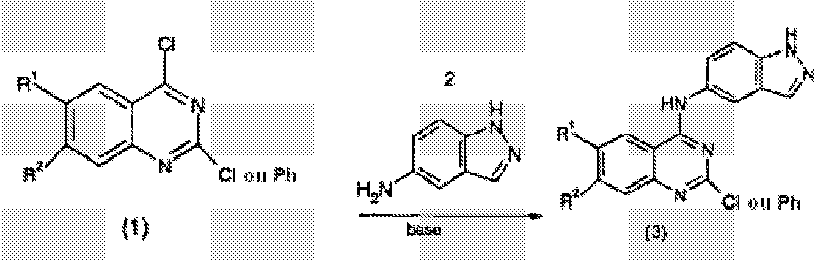 dans lesquelles R¹ et R² peuvent indépendamment être un atome d'hydrogène ou CH₃O-, et Ph est un groupe phényle, et
b) facultativement, faire réagir un composé de formule 3 avec R³NH₂ ou Ar₂NH₂ pour produire un composé de formule 4 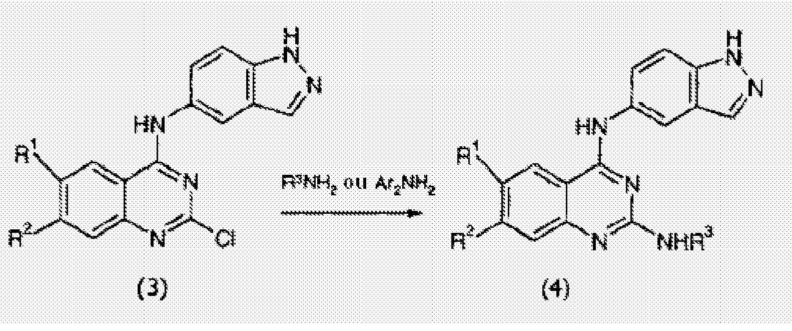
dans lesquelles R³ est CH₃O-CH₂CH₂-, ou un groupe cyclopropyle, et Ar₂ est le 4-fluorophényle ou un groupe pyridyle.

9. Composé selon la formule (3) : 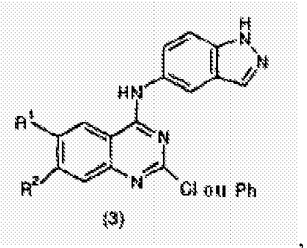 dans laquelle R¹ et R² peuvent indépendamment être un atome d'hydrogène ou CH₃O-, et Ph est un groupe phényle,
ou
la formule (4) : 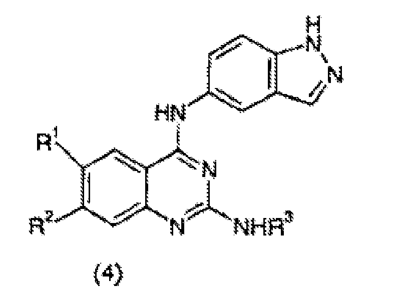 dans laquelle R³ est CH₃O-CH₂CH₂-, ou un groupe cyclopropyle, et Ar₂ est le 4-fluorophényle ou un groupe pyridyle, **caractérisé en ce qu'**il peut être obtenu selon un procédé selon la revendication 8.

10. Composition pharmaceutique, **caractérisée en ce qu'**elle comprend un composé selon l'une quelconque des revendications 1 à 7 ou 9, et un véhicule physiologiquement acceptable.

11. Utilisation d'un composé selon l'une quelconque des revendications 1 à 7 ou 9 pour la préparation d'un médicament destiné au traitement d'une indication impliquant la Rho-kinase.

12. Utilisation d'un composé selon l'une quelconque des revendications 1 à 7 ou 9 pour la préparation d'un médicament destiné aux traitements de l'hypertension, de l'athérosclérose, de la resténose, de l'ischémie cérébrale, du vasospasme cérébral, de la dégénérescence neuronale, d'une blessure de la moelle épinière, d'un cancer du sein, du colon, de la prostate, des ovaires, du cerveau ou des poumons, de troubles thrombotiques, de l'asthme, du glaucome, de l'ostéoporose ou d'un dysfonctionnement érectile.

13. Utilisation selon la revendication 11 ou 12, dans laquelle l'hôte est un être humain.
